# EUROPEAN PATENT APPLICATION

(11) **EP 2 589 301 A1**
(43) Date of publication of application: **08.05.2013**
(21) Application number: 11800225.2
(22) Date of filing: 29.06.2011
(51) Int. Cl.: A23L 1/30, A23L 1/302, A23L 1/36, A61K 36/48, A61P 3/06, A61P 25/28

(54) **INGREDIENT RECONSTITUTED FROM DRY FRUIT AND/OR CAROB**

(30) Priority: 29.06.2010 ES 201031006
(71) Applicant: La Morella Nuts, S.A., 43206 Reus (Tarragona) (ES)
(72) Inventor: RAMÍREZ MARCO, Bartolomé, E-43206 Reus (Tarragona) (ES); REGUANT MIRANDA, Jordi, E-43206 Reus (Tarragona) (ES); ANGLÈS LLAURADÓ, Neus, M., E-43206 Reus (Tarragona) (ES); CABÚS LLAURADÓ, Clara, E-43206 Reus (Tarragona) (ES); MORELLÓ ESTUPIÑÁ, José, Ramón, E-43206 Reus (Tarragona) (ES); ORTEGA OLIVÉ, Nàdia, E-43206 Reus (Tarragona) (ES); TRULLOLS SOLER, Esther, E-43206 Reus (Tarragona) (ES)
(74) Representative: Carpintero Lopez, Francisco
(86) International application number: PCT/ES2011/070469
(87) International publication number: WO 2012/001201

(57) **Abstract**

The present invention describes a reconstituted food ingredient comprising a nut, a carob, a derivative thereof or their mixtures having a low fat content, or a low sugar content, or low fat and sugar content, characterized in that it is enriched with phenolic compounds, and/or sterols and tocopherols originating from nut or carob. The invention also describes a method for preparing said reconstituted food ingredient, as well as its use in the treatment and/or prevention of cardiovascular diseases, vascular diseases, neuronal diseases, neurodegenerative diseases, cancer, inflammatory processes, metabolic syndrome diseases, etc., by means of using this ingredient in the diet. The use of said ingredient in preparing a food composition and various food compositions for human or animal consumption is also described.

## Description

### Field of the Invention

The present invention is comprised within the field of food technology. More specifically, the invention relates to new food ingredients obtained from nuts and/or carob enriched with phenolic compounds, sterols and tocopherols.

### Background of the Invention

Nuts are so called because they all have a common characteristic: their natural composition (without human manipulation) has less than 50% water. They are foods that are very rich in energy, fats, proteins and micronutrients. According to the type of nut, they can also supply substantial amounts of vitamins (especially B group vitamins) or omega-3 (polyunsaturated) fatty acids. Nuts are furthermore very rich in some elements beneficial for health, for example, vitamin E, which has antioxidant properties, rich in most of the B vitamins and rich in a large amount of mineral salts based on metals such as phosphorus, magnesium, copper, iron, etc., making them a very suitable food for vegetarians or vegans. Their calcium content makes them milk substitutes in some cases and ingesting them helps prevent osteoporosis. They are also rich in fibers and ingesting them causes a rapid transit of foods through the intestinal tract. It has been proven that a diet rich in nuts prevents constipation and bowel diseases. The purpose of fiber is to delay sugar absorption, which allows having energy available progressively and for a longer time without it being turned into fats.

Nuts and/or carob contain phenolic compounds, which are bioactive (or functional) substances originating from plant material, are part of the secondary metabolism of plants and provide beneficial effects in humans (Wildman *et al.* 2001) which can act positively on the treatment and/or prevention of diseases in humans and animals, such as cardiovascular diseases, neurodegenerative diseases, metabolic syndrome-related diseases, cancer-related diseases and vascular system-related diseases (Manach *et al.* 2004). Phenolic compounds can be classified into the following subgroups: phenolic acids, flavonoids, lignans, stilbenes and polymerized phenolic complexes (Robards *et al.* 1999). Mostly flavanols and flavanol glycosides are found in nuts, and together they represent between 70-90% of the total phenolic compounds present in the extract (Robards *et al.* 1999). Among the flavanols found in nuts (Milbury *et al.* 2006, Shahidi *et al.* 2007, Monagas *et al.* 2007), catechin, epicatechin, and different procyanidin dimers (the binding of two monomers, generally either catechin or epicatechin) stand out. As a general rule, the glycosylated forms (the binding of flavonol to glucose, galactose, ramnose, rutinose (ramnose+glucose), etc...) of the previous flavanols (Monagas *et al.* 2007) predominate in nuts and carob.

In 1938, Szent-Gyorgy (Szent-Gyorgy *et al.* 1938) suggested the biological activity of flavonoids for the first time, since then a large number of pharmacological effects have been attributed to flavonoids: anti-inflammatory, anti-hepatotoxic, anti-tumor, antimicrobial, antiviral, antiallergenic, enzyme inhibitor, and antioxidant effects and effects on the central vascular system (Mazza *et al.* 2000).

The plant sources listed in the present invention comprise a fat fraction that is made up of various families of compounds, fatty acids being the most known and studied. The functionality of fatty acids is well-known and has been largely proven by means of scientific and intervention studies (Seppänen *et al.* 2002, Dubois *et al.* 2007, Mensink *et al.* 2003). The most important fatty acids for health are unsaturated fatty acids and, specifically, those known as omega-3 and omega-6. These families of polyunsaturated fatty acids have a proven effect in reducing blood low density cholesterol and are accordingly directly related to reducing the risk of cardiovascular diseases. However, there are other components in said fat fraction that also give it greater oxidation stability, as well as beneficial effects for health. Sterols and tocopherols stand out precisely because of that indicated above: for their health-related and technological functionality. Specifically, the effects on health-related functionality are defined as reducing antioxidant activity in different tissues (neuronal, vascular, liver, intestinal, adipose,...), their hypocholesterolemic effect, their anti-inflammatory, antiatherogenic and anti-amyloid activity, and their effects on diseases caused by one or several of these factors: cardiovascular disease, vascular diseases, progression of neurodegenerative diseases, diseases related to cholesterol metabolism, metabolic syndrome, cancer in different organs, skin aging. Other constituents of the fatty phase are, basically, terpene dialcohols, triterpene alcohols, aliphatic alcohols, steroid hydrocarbons, terpene hydrocarbons, volatiles and pigments.

Plant sterols (phytosterols) have become very important in food technology as they are associated with hypocholesterolemic function. Due to the structural similarity between cholesterol and phytosterols, reduced intestinal cholesterol absorption has been seen to occur.

A number of research studies conducted in the last decade have demonstrated that natural vitamin E has antioxidant properties in foods and further delays cell aging as a result of decelerated oxidation, an aspect that is correlated with the decrease in suffering degenerative diseases, and specifically Alzheimer's disease (Vatassery *et al.* 1999).

The beneficial effects of tocopherols and sterols on the human organism are backed by a large number of studies published worldwide which promote their consumption as a functional ingredient.

Dietary fiber, which is present both in nuts and in carob derivatives, is a component that sparks great interest in the nutrition field. A close relationship is established between a fiber-deficient diet and the incidence of specific physiological diseases and disorders, especially in industrialized countries.

Therefore dietary fiber includes polysaccharides, oligosaccharides, lignin, and similar substances. Fiber in turn is divided into two groups based on its water solubility. Soluble fibers are pectins, gums and mucilages. Insoluble fibers are cellulose, hemicellulose, lignin and modified cellulose. Dietary fiber has a heterogeneous mixture of substances having very different physical and chemical properties, which include, in addition to those mentioned: inulin, β-glucans, alginates, carrageenans, xanthans, dextrans, among others. Some of these fibers are obtained in refined form and others are associated with phytochemical compounds (lignans, polyphenols, phytosterols) which, as previously mentioned, also have physiological functionalities (reduced LDL cholesterol, satiating effect,) and synergies have been observed among them (Roberfroid *et al.* 2002; McIntosh *et al.* 2004; Lairon *et al.* 2004).

The nutritional and health advantages of consuming nuts, carob and their derivatives in a balanced diet have been emphasized in the preceding points.

Nevertheless, it must be pointed out that a characteristic of nuts is their high calorie contribution mainly associated with their high fatty acid and carbohydrate content, with a mean calorie contribution of 500 kcal/100 g. In this sense, due to their high calorie content, the consumption of those products and of their derivatives is sometimes restricted or very limited, despite their renowned health properties.

The same occurs with carob, which has a high sugar content. Carobs are edible pods which are used today as fodder in animal food, in the pharmaceutical industry, as well as in the form of flours, gums-additives, in human food. Carob seeds are very rich in mucilages and have the ability to exert a favorable action against mucous membrane inflammations, reducing irritation in both respiratory and intestinal tracts, suitably acting against diarrhea. They also reduce pain resulting from contusions. It is an energy-rich food with a high sugar content, with 50% natural sugar, 10% proteins, as well as minerals such as calcium, iron and phosphorus, and healthy compounds, such as fiber (soluble and insoluble) and phenolic compounds, the consumption of which is also limited despite their beneficial health properties.

Nuts, carob, and their derivatives are widely used in the food industry due to their organoleptic as well as nutritional, functional and technological properties. Nevertheless, depending on the application to be given to these ingredients, there are certain limitations to their application which the present invention seeks to solve. In some food applications it is necessary to adjust the formulations to specific fat or sugar contents for different reasons, whether they are technological reasons or for reasons that enable adjusting the composition to the current European Regulation on nutrition and health claims made on foods (Regulation 1924/2006), as well as a general trend in terms of food and health worldwide. In this sense, the general trend is towards reducing fatty content, especially the saturated fatty acid content, and sugar content, as well as reducing salt. An alternative proposed in the state of the art for increasing their consumption, for example in pastry making, in sauces, diet food, snacks, products derived from grains (cookies, breakfast cereals,...etc.), is based on reducing at least in part their calorie contribution associated with the intrinsic fat and/or sugar content. However, this alternative has serious drawbacks: a number of compounds with health and/or nutritional properties are removed together with the extracted fat and/or sugar fractions, their bioaccessibility and therefore their health properties being substantially reduced. Furthermore, the usual processes for preparing them negatively affect the organoleptic, aromatic and/or texture properties, sometimes causing consumers to reject them.

One way of overcoming these drawbacks could be to supplement nuts and/or carob with functional compounds, taking into account that adding various bioactive compounds to various foods and food ingredients to provide products with functional properties is well known in the state of the art. However, many of these bioactive compounds have a negative effect on foods and food ingredients because they technologically and sensorially modify them and divert them from the original matrix. Furthermore, many bioactive compounds do not retain their bioactivity once they are processed and ingested. In order to assure their bioactivity, for example, in the specific case of phenolic compounds and sterols and tocopherols, it is known in the state of the art that said compounds must be bioavailable. Bioavailability can be measured by means of bioaccessibility using *in vitro* methodologies. Bioaccessibility is understood as "the property" that the fraction of bioactive compounds (polyphenols, sterols and tocopherols) released from the food matrix that is ingested and digested can potentially be absorbed in the gastrointestinal tract. This property is measured by means of an *in vitro* digestion method.

Therefore and in view of the foregoing, it would be desirable to provide new processed ingredients based on nuts and/or carob that are low in fats and/or sugars and rich in functional bioactive compounds, thereby increasing the consumption possibilities thereof, that do not sensorially alter the products, with good organoleptic properties and that assure the stability and activity of the functional compounds once they are processed and ingested.

### Description of the Invention

In one aspect, the present invention provides a reconstituted ingredient comprising a starting product selected from the group consisting of: nuts, carob, nut derivatives, carob derivatives and mixtures thereof, having (i) a low fat content, or (ii) a low sugar content, or (iii) a low fat and sugar content, characterized in that it is enriched with phenolic compounds, sterols and tocopherols originating from nut or carob.

In a particular embodiment, the starting product is a nut. In the context of the present invention the term nut refers to any fruit, of any origin or source, which can be raw, toasted, boiled, roasted, fried, with or without skin, the moisture content of which is less than 50%. Illustrative examples of nuts that can be used in the present invention are: hazelnut, almond, peanut, walnut, pistachio, cashew, chestnut, pine nut, macadamia nut, Brazil nut, pecan, tiger nut, dried fresh fruit, gevuina (Chilean hazelnut), quinoa, chia, among others, and seeds such as sesame, sunflower, linseed, pumpkin, safflower, poppy, cocoa, coffee, broad beans and spices, as well as grains.

In another particular embodiment, the starting product is a carob derivative. In the context of the present invention, the term carob derivative refers to any product that can be obtained from carob, for example carob powder. In another particular embodiment, the starting product is a nut derivative. In the context of the present invention, the term nut derivative refers to any product that can be obtained from nut, for example, granules, flour, paste, skin or oil, among others. In the case of oil, it has a low saturated fraction and is rich in sterols and tocopherols and unsaturated fraction.

These derivatives are obtained typically from the whole carob or the whole nut, or from any of their parts or a combination thereof, such as the legume, the seed, the pod, the flesh, the shell, the skin. Methods for obtaining these derivatives are well known for the person skilled in the art and generally comprise at least one physical, mechanical or chemical process step, such as cleaning, separating the desired part of the nut or carob, grinding, sieving, purifying, drying, lyophilizing, milling, pressing, extracting with solvents or with supercritical fluids, toasting, frying, extracting with microwaves, extracting with ultrasounds, etc.

The terms low in fat, low in sugars and low in fat and sugars, in relation to the starting product, refer to the fact that the fat, sugar or fat and sugar content present in a starting product (nut, carob, nut derivative, carob derivative or in mixtures thereof) is lower with respect to the fat, sugar or fat and sugar content, respectively, than what said raw material has originally as a characteristic inherent thereto, or after being obtained in a conventional manner in the case of derivatives.

The reduction of the fat content of a raw material, whether it is a nut, carob, nut derivative, carob derivative or of a mixture thereof, with respect to its initial content is carried out by means of a separation process, preferably extraction. Said extraction can be a physical extraction by means of pressing, it can be an accelerated, conventional, or supercritical extraction with solvents and in conditions known for a person skilled in the art. In this sense, the extraction used for reducing the fat content can be carried out by means of different methods, some of which are mentioned in the literature (Marrone *et al.* 1998; Amarowicz *et al.* 2005). Another alternative that can be used in the case of reducing the saturated fat fraction content of a nut oil can be, by way of example, the use of fractionation process by means of different techniques known by any person skilled in the art.

The sugar content of a raw material, whether it is a nut, carob, nut derivative, carob derivative or of a mixture thereof, can be reduced with respect to its initial content by means of accelerated and/or conventional extraction. The extraction conditions used for reducing sugar content are described in the following articles (Kumazawa *et al.* 2002; Garcia-Noguera *et al.* 2010).

The fat and sugar content of a raw material is reduced by means of at least two alternatives: i) first a total or partial fat content reduction, and then a total or partial sugar content reduction, ii) or a total or partial sugar content reduction, and then a total or partial fat content reduction. The methods cited above are applied in both cases.

These products with a lower fat, sugar or fat and sugar content with respect to the corresponding raw material are herein referred to as starting products.

As discussed in the background of the invention, in the process for obtaining the starting products, the total or partial fat content reduction involves bioactive compound content reduction in the fat fraction (sterols and tocopherols, among others). The same occurs in the process for total or partial sugar content reduction; many bioactive compounds such as the phenolic compounds that are normally bound to sugars are removed in this step. (Ortega *et al.* 2010 submitted). Many volatile compounds which are characteristic of nuts are also removed in the process for reducing the fat fraction. This drawback can interfere in the bioaccessibility of the bioactive compounds and in the subsequent acceptability of the resulting product.

The reconstituted ingredient of the invention can be enriched with phenolic compounds, and/or with sterols and tocopherols and is obtained by incorporating these bioactive compounds to the starting product. In the context of the present invention, rich must therefore be understood as the ingredient having a content made up of these bioactive compounds exceeding the original content present in the starting product. These compounds preferably originate from nut, carob, from their derivatives or mixtures thereof as explained below. In a particular embodiment, the compounds are added in the form of an extract rich in phenolic compounds and/or a fat extract rich in sterols and tocopherols. These extracts can be obtained from nut or carob, i.e., either from the whole nut or from the whole carob or from any of their parts or derivatives or from mixtures thereof.

It is interesting to use an extract rich in phenolic compounds and a fat extract rich in sterols and tocopherols (among other compounds of interest) in the present invention for the purpose of being able to enrich the starting product using a reduced volume.

An extract rich in phenolic compounds or phenolic extract useful for putting the present invention into practice originates from a nut, carob or of mixtures thereof and can be obtained commercially or by means of known extraction methods (Papagiannopoulos *et al.* 2004; Kumazawa *et al.* 2002; Monagas *et al.* 2007; Yu *et al.* 2005; Moure *et al.* 2006; Kamath *et al.* 2007; Counet *et al.* 2003; Ortega *et al.* 2008; Shahidi *et al.* 2007).

The process for obtaining the phenolic extract can consist of the following steps:
a) Starting material pre-treatment(s): this step facilitates the subsequent extraction of the phenolic compounds as it enhances the accessibility of the extraction solvents to the inaccessible plant tissue fractions. The pre-treatments applied according to the consistency of the starting material can consist of hydrolysis (acid or alkaline), washing, enzymatic treatment, maceration, treatment with ultrasounds, microwaves (Ballard *et al.* 2010), a step for lyophilizing and/or defatting the initial material at temperatures ranging between 20°-C and 100°-C.
b) Extraction: can be performed by means of using fluids having a specific polarity and preferably for food use, such as alcohols, ketones, water and/or mixtures thereof for example; as well as by means of hydrocarbons and CO₂ under supercritical conditions. The extraction is performed at a temperature no greater than 100ºC for the purpose of minimizing degradation of the phenolic compounds; the pressure of the process will be comprised between atmospheric pressure and 60 MPa (600 bar), depending on whether the extraction is an accelerated, conventional or supercritical extraction, for a period of time comprised between 15 min and 100 h. Alternatively, the extraction of phenolic compounds can be carried out by means of using ion exchange resins or membranes (Sarmento *et al.* 2006).
c) Post-treatment(s): a post-treatment can be applied to the phenolic extract obtained before being used in the formulation of the reconstituted ingredient whether it is to facilitate its incorporation and solve technological problems: sensorial or dispersion problems, or for assuring its stability, bioaccessibility and therefore functionality with regard to its health benefit. Spray drying or freeze drying can be carried out for that purpose; alternatively, the extraction solution can also be concentrated without needing to reach dryness, and the fluid can also be incorporated as indicated below. In a particular embodiment, encapsulation of the phenolic extract can be carried out before being incorporated into the starting ingredient by means of techniques known by a person skilled in the art, such as, for example, the use of a spray-dryer, liposomes, fluidized bed, among others.

The phenolic extract obtained is characterized in that it has a phenolic content ranging between 5-60% expressed on the basis of a catechin standard by means of the Folin-Ciocalteu method (Singleton *et al.* 1998) as well as by the presence of simple sugars, soluble and insoluble fiber and moisture.

In a particular embodiment of the invention, said extract has the appearance of a reddish-brown hygroscopic powder that is partially soluble in water at a temperature of 20ºC and soluble in alcohol.

A fat extract rich in sterols and tocopherols useful for putting the present invention into practice originates from the nut or from mixtures thereof and can be obtained commercially or obtained by means of extraction methods known by a skilled person (Kornsteiner *et al.* 2006).

The process for obtaining the fat extract rich in sterols and tocopherols can consist of the steps following:
a) Pre-treatment(s): the nut or derivatives thereof can be subjected to a pre-treatment such as ultrasounds, microwaves, among others, for the purpose of increasing the yield of obtaining the fat extract rich in sterols and tocopherols.
b) Extraction: the process for obtaining the fat extract rich in sterols and tocopherols can be obtained by means of conventional distillation with or without reduced pressure or molecular distillation (ES 2 128 400). It can particularly be carried out under supercritical conditions using a fluid, such as CO₂, for example, as a solvent. Said supercritical extraction process takes place at temperatures less than 60ºC and at pressures between 8 and 60 MPa (80 and 600 bar). In some cases, a cascade of pressures can be applied between the splitters for the purpose of obtaining a fractionation of the extract. The pressure and temperature conditions, the CO₂ mass:sample mass ratio, the pre-treatment of the initial sample, the filler content, etc., are parameters susceptible to being varied according to the nut or derivatives. It is also possible to obtain the volatile fraction of said nut or derivatives during the process for obtaining the fat extract rich in sterols and tocopherols. Said extraction can be favored by means of ultrasounds. An accelerated extraction can also be carried out, as can an extraction be performed at atmospheric pressure by means of conventional methods with organic solvents (Shahidi *et al.* 2008a).

The yield of the extraction for obtaining a fat extract rich in sterols and tocopherols ranges, for example, between 3000 and 16000 mg sterols and tocopherols per kilogram of fatty phase obtained using extraction with supercritical CO₂ (Lu *et al.* 2007). These values are a function of the nut or derivative thereof.
c) Post-treatment(s): a post-treatment can be applied to the fat extract rich in sterols and tocopherols obtained before being used in the formulation of the reconstituted ingredient whether it is for facilitating its incorporation (and solving technological problems: dispersion); assuring its stability and therefore its functionality concerning health. Encapsulation of the fat extract rich in sterols and tocopherols can be carried out for that purpose. Encapsulation of the fat extract rich in sterols and tocopherols can be carried out by means of using vehicles or excipients, such as for example, whey proteins, dextrins, acacia gum, (Laine *et al.* 2010a). The yield of encapsulating can range between 1-85% (Torchiuli et al., 2005; Somchue et al., 2009; Fuchs et al., 2006), and it must be pointed out that this technique is used in the event that direct dispersion is not possible for technological reasons or because of a loss of functionality, given that the use of this technique involves diluting the sterol and tocopherol content of the post-treated fatty phase extract with respect to the original extract.

The fat extract rich in sterols and tocopherols, hereinafter also fat extract, is characterized by means of gas chromatography (according to the method disclosed in Leo *et al.*, 2005) and has sterol and tocopherol values between 1 and 20 g per kg of extract obtained.

The present invention contemplates the possibility of obtaining extracts from mixtures of different types of raw materials as a function of the concentrations of phenolic compounds or of sterols and tocopherols that are to be obtained in the resulting extracts.

The reconstituted ingredient is obtained from the corresponding starting product low in fat, low in sugars or low in fats and sugars by the incorporation therein of at least a phenolic extract and/or a fat extract rich in sterols and tocopherols obtained from nut, carob or mixtures thereof as described above.

In obtaining the reconstituted ingredient of the invention the amount of extract added to the starting product can vary within wide ranges. Said amounts are comprised between the minimum amount necessary for the reconstituted ingredient to have a higher phenol and/or sterol and tocopherol content with respect to the starting product, up to the maximum amount that can be incorporated in said product. These amounts can vary, for example, as a function of the reconstituted ingredient to be prepared in each case, of the nature and characteristics of the extract and of the starting product, etc. In a particular embodiment, the amount of extract must be such that indicated reference values which allow making health claims are reached in each case.

In principle, one or most phenolic extracts and one or more fat extracts rich in sterols and tocopherols, which are in different proportions in relation to one another and in relation to the starting ingredient, can be used.

In a preferred embodiment, a phenolic extract and a fat extract are incorporated in the starting product. The incorporation thereof can be done either independently and separately for each extract or simultaneously and prior to combining the extracts. There are various techniques that can be used in the present invention for incorporating extracts. Some applicable techniques are listed by way of illustration:
(i) dispersion and
(ii) encapsulation;
(iii) gel/emulsion

i) Dispersion comprises the direct addition of the extract (of each type of extract independently or of the mixture thereof) to the starting product followed by homogenization, suspension, immersion or direct dissolution thereof. This option is suitable in those cases in which the direct addition contributes desired organoleptic modifications by the ingredient or it does not entail unwanted technological changes.
ii) Encapsulation can be suitable when the matrix of the starting ingredient is a solid (whole nut or granules) that has been defatted and/or has a total or partial sugar reduction. In this case, it comprises the direct addition of the extract (of each type of extract independently or of the mixture thereof) to the starting product and the application of a coating. In a particular embodiment, said process comprises applying an edible film-forming composition comprising the extract and allowing its encapsulation on the starting product. The nature and the properties of the phenolic compound, sterol and tocopherol extracts, and of the starting product determine the nature and amount of the film-forming layer in each case. In a particular embodiment, generally any conventional edible polymer, such as cellulose derivatives, such as hydroxy methyl celluloses (HPMC), methyl celluloses (MC); acacia gum, protein derivatives (whey), lipid derivatives, can be used as a film-forming agent, optionally together with at least one excipient. The amount of composition necessary and of film-forming coating which is obtained in each case is a function of the amount of the extract to be encapsulated.
iii) In a particular embodiment, emulsion/gel can be used in the event that it is necessary to incorporate any type of ingredient in the starting product for providing texture to the combination or for completely or partially substituting the fat and/or sugar that has been removed from the raw material. In this case, the use of emulsions and/or gels as a technique for performing the prior mixture of the phenolic compound, sterol and tocopherol extracts for vehiculating them in the starting product by means of incorporating such extracts in a hydrocolloid solution (based on proteins, carbohydrates, lipids, gums, fibers, etc.), is contemplated.

By way of illustration, as a function of the physicochemical properties of the starting ingredient and of the characteristics of the extracts, such extracts can be incorporated by means of different modes of application, some of which are exemplified in the following table:

| Starting product | Polyphenolic extract | | A fat extract rich in/ fatty phase with a high concentration of/rich in sterols and tocopherols | |
|---|---|---|---|---|
| | Characteristics | Mode of incorporation | Characteristics | Mode of incorporation |
| Fluid (at least 30% fat) | Powder | dispersion | Fluid | Dispersion |
| Fluid | Powder | dispersion | Powder | Dispersion |
| Fluid | Fluid | dispersion | Fluid | Dispersion |
| Solid (flour, powder) | Powder | dispersion | Powder | Dispersion |
| Solid (flour, powder) | Powder | dispersion | Fluid | Dispersion/at omization |
| Solid (granules, whole) | Powder | encapsulation | Powder | Encapsulation |
| Solid (granules, whole) | Powder | encapsulation | Fluid | Encapsulation |

The advantages of the reconstituted ingredient, among others, is that it is a natural food ingredient low in fat and/or sugar and with a higher density in phenolic compounds, sterols and tocopherols with respect not only to the starting product, but optionally with respect to the corresponding starting raw material. This aspect makes it a new ingredient very useful in formulations of foods which do not wish to dispense with the use of nuts and/or carob or mixtures thereof, but which does wish to prevent incorporating a higher fat and/or sugar content. In the field of food, the range of ingredients from nuts and/or carob applicable in food compositions with a limited fat and/or sugar content but with the need to have a specific nut content (for example in accordance with European Regulation nutritional profiles or with general food industry fat, sugar and salt-reducing trends) is thereby increased.

The reconstituted ingredient of the invention based on nut and/or carob can at the same time have a higher concentration of phenolic compounds and/or sterols and tocopherols than the original raw material (nut, carob or their derivatives) due to the partial removal of the fat and, optionally, of the sugar and due to the subsequent incorporation of phenolic compounds, sterols and tocopherols originally from the actual raw material in variable amounts.

As a function of the method for concentrating the sterol and tocopherol compounds, an extract with a high concentration of such compounds as well as with a high concentration of aromatic volatile compounds characteristic of the actual nut from which they are obtained can be obtained. In this case, it is interesting to incorporate, for example, this extract in the same starting product from which the extract originates, whereby achieving a reconstituted ingredient according to the invention that is low in fat and/or sugars but rich in bioactive compounds characteristic of the raw material in question, and with the additional advantage of having a better aromatic quality than that of the starting product or raw material.

Phenolic compound, sterol and tocopherol extracts can also be incorporated in a starting product rich in unsaturated fatty acids and low in or with an absence of saturated fatty acids, such as an oil for example, whereby the use of this dual system based on phenolic compounds, tocopherols and sterols protects unsaturated fatty acids with a faster tendency to oxidize against oxidation.

The reconstituted ingredient of the invention further has the advantage of being functional and its functionality can be controlled as a function of the amount and the type of extract with which the starting product which can furthermore have been obtained from the same source is enriched. In this sense, although on one hand a fraction of the functional compounds present in the original raw materials is removed in the process for defatting and/or for reducing the sugar, on the other hand these materials (starting products) are enriched with phenolic compounds and/or with sterols and tocopherols originating from incorporating a concentrated extract the vehiculation of which in the corresponding food matrix favors or assures its bioaccessibility in the organism. If desired, the reconstituted ingredients of the invention can maintain the same structure and consistency as the starting product and a sensorial profile typical of a nut and/or carob when desired, reducing the total fat content with respect to the raw material and the calorie contribution (fat and/or sugar content) but without renouncing the desired sensorial characteristics of each type of raw material (nut, carob or derivatives thereof).

In this sense, the invention proposes reducing said calorie contribution, mainly associated with the fat and/or sugar content, without at the same time depriving said ingredient of the organoleptic component that characterizes it, and of the bioactive substance content of interest for health such as: unsaturated fatty acids, sterols and tocopherols present in the fat fraction, as well as phenolic compounds, mainly of the skin.

An advantage of the present invention consists of promoting the use or consumptions of this type of reconstituted ingredient obtained from nuts and/or carob as an alternative to the usual raw materials, which many times cannot be used or consumed due to their high fat content, or their high sugar content, or both, due to technological and nutritional profile as well as sensorial profile issues. In this sense, this patent provides a reconstituted ingredient having a density in bioactive substances and/or technological substances of interest greater than the starting product and/or greater than the raw material from which the starting product originates.

Physiologically speaking, the phenolic compounds and tocopherols and sterols provide functionality (for health) through different biological mechanisms, as indicated in the background section. Another advantage of the present invention consists of assuring the bioaccessibility of the bioactive compounds of both extracts once the fat and/or sugars typical of the starting nut and/or carob have been reduced. In this sense, the ingredient of the invention has antioxidant, anti-inflammatory, hypocholesterolemic and hemostatic regulation capabilities. As a result, the ingredient of the invention is useful in the treatment and/or prevention of cardiovascular diseases, vascular diseases, neuronal diseases, neurodegenerative diseases, cognitive disorders, cancer, skin diseases, inflammatory processes, hypercholesterolemia, kidney diseases, digestive system diseases, bone diseases, metabolic syndrome and immune system diseases, among others, in a human or animal. The ingestion thereof by a mammal, human or animal, has beneficial effects on health, on the cognitive function, vascular function, immunological function, among others.

In a particular embodiment, the ingredient of the invention has both extracts rich in phenols and sterols and tocopherols incorporated therein, providing said reconstituted ingredient with a synergistic effect in terms of both physiological and technological functionality. This synergy is due to different factors: the compatibility between the physiological role played by each of said compounds, as well as the protection against degradation they exert with respect to one another, as well as the enhancement of the bioaccessibility of the phenolic compounds in the organism as a result of their vehiculation in the mixture in the reconstituted ingredient, and in turn, the sterols and tocopherols can carry out functionality as described above. The mixture of the two types of extracts prior to their addition to the matrix low in nut and/or carob fat and/or sugar, which are found naturally in nuts/carob, provides a synergistic mixture with benefits on human or animal health.

One of the technological functionalities is due to the lower fat content with respect to the raw material. In a particular example of the invention, the reconstituted ingredient can be suitable in those applications in which the effect of the migration of fat in the end product incorporating the reconstituted ingredient is to be reduced.

The reconstituted ingredient has an additional technological advantage relating to its high stability in subsequent processing steps. The incorporated extracts from the actual nut and/or carob are protected while at the same time if desired, the sensorial profile of the nut and/or carob of the end product is enhanced and the capability of such raw materials to be used in food compositions is increased.

Therefore, in another aspect the invention relates to the use of the reconstituted ingredient of the invention in preparing a food composition. Food ingredients and foods both for human and animal consumption are encompassed within the food compositions in the present invention. These foods and food ingredients are an additional aspect of the present invention.

Said foods or food ingredients which are another aspect of the invention can include, for example, among others, cereal fillings, sandwich-type cookie fillings, wafer-type fillings, for spreadable creams, or dip-in type creams, fillings for mass-produced pastries (for example croissants, *ensaimadas* (Mallorcan cakes), etc.); chocolates, nuts themselves; snacks, muesli, powders for instant drinks, non-dairy drinks, desserts, breads, dairy products, ice creams, pasta fillings, cereal fillings, pizzas, sauces, nougat candies, and dietetic products in general, among other particular applications of the reconstituted ingredient of the invention.

Illustrative examples of the invention which are explained to better understand the invention and which in no case should be considered a limitation of the scope thereof are provided below.

### EXAMPLES

### Example 1. Hazelnut paste low in fats and rich in sterols, tocopherols and phenolic compounds characteristic of its matrix

A hazelnut paste was obtained by means of grinding hazelnuts. The hazelnut paste is characterized in that it is a fluid fat suspension the particles of which have a grain size distribution mostly between 60-30 µm.

The starting defatted matrix, i.e., starting product, for preparing the reconstituted ingredient was then obtained from 100 g of hazelnut paste which were defatted by adding hexane at a ratio of 1:10 (w/v). The mixture was kept under stirring for 15 minutes. The supernatant was separated by centrifugation. The partially defatted hazelnut paste was obtained once the supernatant was removed. The yield of defatting by means of this process is an approximately 15% fat fraction.

The process for obtaining the fat extract rich in sterols and tocopherols was carried out under supercritical conditions using CO₂ as a solvent.

Said supercritical extraction process was started with a 100 g of hazelnut paste which were mixed with a dispersing agent (inert) at a ratio of 1:1. The extraction took place at 50ºC and at a pressure of 12 and 8 MPa (120 and 80 bar). On average, 5 grams of a fat extract rich in sterols and tocopherols were obtained characterized in that they had a sterol and tocopherol content of 12 g per kilogram of extract. This sterol and tocopherol content was determined by means of gas chromatography (Leo *et al.* 2005).

A phenolic compound extract was obtained from 10 g of nut skin to which there were added 80:20 (v:v) acetone:water at a ratio of 1:10 (w:v) and the resulting mixture was kept under stirring for 15 minutes at 200 rpm. After this time elapsed, the supernatant was separated by centrifugation for 10 min at 8ºC. The organic solvent fraction was removed by means of distillation and the aqueous residue was lyophilized for obtaining a phenolic extract in the form of powder with a 12% extract yield with respect to the starting skin.

Said phenolic extract was characterized in that it had a total phenol content of about 30%, expressed with catechin, the determination of which is performed by means of the Folin-Ciocalteu method (Singleton *et al.* 1998).

The reconstituted ingredient was formulated from the composition of the starting ingredient, and of the phenolic compound extracts and a fat extract rich in sterols and tocopherols. Five grams of a fat extract rich in sterols and tocopherols and 10 g of polyphenolic extract were added to the partially defatted hazelnut paste (85 g). The fat extract rich in sterols and tocopherols and the phenolic compound extract were incorporated in the starting ingredient by means of direct dispersion.

A reconstituted ingredient which can be applied in those food formulations in which it is necessary to use nuts but which wish to dispense with the fatty part without dispensing with the compounds of interest, such as sterols and tocopherols, and which further wishes to enhance the benefit of phenolic compounds because their bioaccessibility is assured by means of the described preparation process, was obtained. The bioaccessibility of the phenolic compounds of the reconstituted ingredient has been evaluated by means of *in vitro* digestion.

### Example 2. Carob powder low in sugars and rich in sterols and tocopherols and phenolic compounds of carob

One hundred grams of carob powder were weighed and an aqueous solution was added to it at a ratio of 1:20 (w/v). The resulting mixture was kept under stirring for 10 hours at room temperature. The supernatant was separated and disposed by means of centrifugation at 10000 rpm for 10 min. The yield of removing sugars was 30%.

On the other hand, a phenolic compound extract was obtained from 100 g carob powder. A conventional extraction was carried out with a mixture of acetone:water at ratios of 80:20 (v/v) at room temperature. The extraction step was carried out by subjecting the mixture to maceration for 1 h, separating the solvent and the spray drying step. The phenolic extract which was obtained with a yield of 12% on the basis of the starting carob powder was characterized in that it had a total phenol content of about 30%, expressed with catechin, the determination of which was performed by means of the Folin-Ciocalteu method.

The fat extract rich in sterols and tocopherols was obtained from almond using CO₂ under supercritical conditions, at temperatures less than 60ºC and at pressures of 10 MPa (100 bar) for 8 hours. The fat extract rich in sterols and tocopherols had a concentration of 10 g of sterols and tocopherols per kilogram of extract. Said extract was subjected to encapsulation by means of using a spray-dryer using maltodextrins as the carrier agent. The yield of encapsulating the fat extract rich in sterols and tocopherols was 10%.

Fifteen grams of the carob phenolic extract and 10 g of the fat extract rich in sterols and tocopherols, previously encapsulated, were added to the carob powder low in sugars (75 g) by means of dispersion, obtaining a reconstituted ingredient. This reconstituted ingredient was characterized in that it was a homogenous and functional mixture. A technical improvement of the reconstituted ingredient is its suitable sugar composition for use in foods with nutritional profiles suitable for those indicated in laws regulating foods with nutritional claims and assuring the bioaccessibility of the extracts for obtaining health effects.

### Example 3: Nut granules low in fat and rich in sterols and tocopherols and polyphenolic extract.

Hazelnut granules were obtained by means of granulating and subsequently hazelnut sifting. The hazelnut granules were characterized in that they had a grain size distribution between 6-10 mm.

Partial defatting of 100 g of nut granules was carried out by means of a conventional extraction with hexane at a ratio of 1:10 (w/v). As in Example 1, the mixture was kept under stirring for 15 minutes. The supernatant was separated by centrifugation. The partially defatted hazelnut paste was obtained once the supernatant was removed. The yield of defatting by means of this process was an approximately 15% fat fraction. Alternatively, defatting can be carried out in this same example obtaining the same yield of a 15% fat fraction by means of suitable extraction conditions with CO₂ under supercritical conditions.

The fat extract rich in sterols and tocopherols was obtained from subjecting the nut to an extraction process under supercritical conditions using liquid CO₂ as a solvent. Said supercritical extraction process takes place at temperatures less than 60ºC and at pressures between 8 MPa (80 bar). The fat extract rich in sterols and tocopherols had a concentration of 12 g of sterols and tocopherols per kilogram of extract.

The phenolic compound extract was obtained as in the preceding example under conventional conditions using a mixture of organic solvent with water. In this specific case 80:20 (v/v) acetone:water is added to the nut skin at a ratio of 1:10 (w:v) and is kept under stirring for 15 minutes. After this time has elapsed, the supernatant is separated by centrifugation. The organic solvent fraction is removed from the supernatant by means of vacuum evaporation and the aqueous residue is lyophilized for obtaining the phenolic fraction in the form of powder with a yield of 20% with respect to the starting nut granules. The phenolic fraction that is obtained is characterized in that it has a total phenol content of about 30%, expressed with catechin, the determination of which is performed by means of the Folin-Ciocalteu method.

An emulsion was prepared with both extracts: phenolic extract and fat extract rich in sterols and tocopherols and lecithin at a ratio of 0.5:1:0.1 (by weight). Then 8 g of the emulsion obtained from both extracts is incorporated in 100 g of defatted hazelnut granules by means of trickling in a drum, and the coating is subsequently applied and dried.

### Example 4: Nut and cocoa cream low in fat and sugar based on partially defatted nut flour and rich in a fat extract rich in sterols and tocopherols and in phenolic compound extract and carob powder low in sugar

In the following example, the reconstituted ingredient is applied in a spreadable cocoa cream. It was based on a spreadable cocoa cream type formulation in which the basic nut ingredient was replaced with
- partially defatted nut flour and rich in sterols and tocopherols and phenolic compound extract (as the flour obtained in Example 1), and
- carob powder low in sugar (as that obtained in Example 2, but without incorporating the extract rich in sterols and tocopherols or the phenolic compound extract).

## Claims

1. Reconstituted food ingredient comprising a starting product selected from the group consisting of nuts, carob, nut derivatives, carob derivatives and mixtures thereof having (i) a low fat content, or (ii) a low sugar content, or (iii) a low fat and sugar content, **characterized in that** it is enriched with phenolic compounds, sterols and tocopherols originating from nut or carob.

2. Reconstituted food ingredient according to claim 1, wherein the starting product is a raw, toasted, boiled, roasted, fried nut, with or without skin, selected from the group consisting of hazelnut, almond, peanut, walnut, pistachio, cashew, chestnut, pine nut, macadamia nut, Brazil nut, pecan, tiger nut, dried fresh fruit, gevuina, quinoa, chia, sesame, sunflower, linseed, pumpkin, safflower, poppy, cocoa, coffee, broad beans, spices, grains and their mixtures.

3. Reconstituted food ingredient according to claim 1, wherein the starting product is a carob derivative, particularly carob powder.

4. Reconstituted food ingredient according to claim 1, wherein the starting product is a nut derivative, particularly granules, flour, paste, skin or oil.

5. Method for preparing a reconstituted food ingredient according to any one of the preceding claims which comprises incorporating (i) an extract rich in phenolic compounds or (ii) a fat extract rich in sterols and tocopherols or (iii) a mixture of both, said extracts being obtained from nut or carob, to a starting product selected from a nut, a carob and a derivative thereof having (i) a low fat content, or (ii) a low sugar content, or (iii) a low fat and sugar content.

6. Method for preparing a reconstituted food ingredient according to claim 5, wherein the phenolic extract is **characterized in that** it has a phenolic content ranging between 5-60% expressed on the basis of a catechin standard by means of the Folin-Ciocalteu method.

7. Method for preparing a reconstituted food ingredient according to claim 6 or 5, wherein the fat extract rich in sterols and tocopherols has a sterol and tocopherol content between 1 and 20 g per kilogram of extract determined by means of gas chromatography.

8. Method for preparing an ingredient according to claims 5 to 7, wherein the phenolic extract, the fat extract rich in sterols and tocopherols, or both extracts are subjected to an encapsulation step before being incorporated into the starting product.

9. Method according to any one of claims 5 to 8, wherein the amount of phenolic extract added to the starting product is the amount necessary for the reconstituted ingredient to have a higher phenol content with respect to the starting product and/or optionally to the starting material from which the starting product is obtained and/or the amount of a fat extract rich in sterols and tocopherols added to the starting product is the amount necessary for the reconstituted ingredient to have a higher sterol and tocopherol content with respect to the starting product and/or, optionally, higher with respect to the starting material from which the starting product is obtained.

10. Method according to any one of claims 5 to 9, wherein incorporating the phenolic extract, the fat extract rich in sterols and tocopherols or the mixture of both can be done by means of the dispersion, encapsulation or gel/emulsion technique.

11. Method according to claim 10, wherein the dispersion technique comprises at least one of the following steps:
direct addition of the extract to the starting product;
homogenization;
suspension;
immersion or
direct dissolution thereof.

12. Method according to claim 10, wherein the encapsulation technique comprises applying an edible film-forming composition comprising the phenolic extract, the fat extract rich in sterols and tocopherols or a mixture thereof on the starting product.

13. Method according to claim 10, wherein the emulsion or gel technique comprises incorporating the mixture of phenolic extracts and a fat extract rich in sterols and tocopherols to a hydrocolloid solution followed by vehiculating same in the starting product.

14. Reconstituted food ingredient according to any one of claims 1 to 4, for use in the treatment and/or prevention of cardiovascular diseases, vascular diseases, neuronal diseases, neurodegenerative diseases, cognitive disorders, cancer, skin diseases, inflammatory processes, hypercholesterolemia, kidney diseases, digestive system diseases, bone diseases, metabolic syndrome and immune system diseases.

15. Use of the reconstituted food ingredient according to any one of claims 1 to 4 in preparing a food composition.

16. Food composition for human or animal consumption comprising the reconstituted food ingredient according to any one of claims 1 to 4.
